(19) Europäisches Patentamt
European Patent Office
Office européen des brevets

(11) EP 2 213 221 A1

(12) EUROPEAN PATENT APPLICATION

(43) Date of publication:
04.08.2010 Bulletin 2010/31

(51) Int Cl.:
A61B 1/01 (2006.01)        A61B 1/005 (2006.01)

(21) Application number: 10004087.2

(22) Date of filing: 30.10.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 29.11.2007 JP 2007308830

(62) Document number(s) of the earlier application(s) in
accordance with Art. 76 EPC:
08018968.1 / 2 064 984

(71) Applicant: Olympus Medical Systems Corp.
Tokyo 151-0072 (JP)

(72) Inventors:
• Takahashi, Kazuhiko
  Hachioji-shi
  Tokyo 192-8512 (JP)
• Nakamura, Toshio
  Hachioji-shi
  Tokyo 192-8512 (JP)

(74) Representative: Schmidt, Steffen
Wuesthoff & Wuesthoff
Patentanwälte
Schweigerstrasse 2
81541 München (DE)

Remarks:
This application was filed on 16-04-2010 as a
divisional application to the application mentioned
under INID code 62.

(54) **Therapeutic device system and manipulator system**

(57) A therapeutic device system is provided with a control parameter section (8) for calculating a control parameter for adjusting a drive amount of a manipulator (2) with respect to a manipulation signal on the basis of curved state information on an endoscope insertion section (27), and changing the old control parameter, and when a state of a curve of the endoscope insertion section (27) has changed, a control parameter conforming to the change is calculated to change the old one, whereby the same manipulation operation of the operator enables the same operation of the manipulator (2) to be performed at all times.

FIG. 1

EP 2 213 221 A1

**Description**

The present invention relates to a therapeutic device system and a manipulator system including an active therapeutic device to be inserted into an insertion section of an endoscope apparatus.

In general, a manipulator in which a plurality of rods are universal-joint-connected to each other by means of articulation sections, and which can be freely curved is known as one of the master-slave type arm robot. For example, there is a manipulator used as a therapeutic device which is inserted into a therapeutic device hole of an endoscope insertion section of an endoscope apparatus to be used for various treatments.

Various therapeutic devices corresponding to uses such as an electric-cautery, forceps, and the like are attached to the distal end section of the manipulator.

The inside of each of rods of this manipulator is connected to a drive wire, and the articulation section is bent by pulling the wire. When the wire pulling operation is performed, at least two wires are used for one articulation section, i.e., one degree of freedom, and hence twice as many wires as the number of articulation sections are arranged. By adjusting the traction amount of each of the wires, the positional posture can be changed as desired.

Normally, the wires of the manipulator are arranged in the therapeutic device insertion section. In the therapeutic device insertion section, wires which are inserted into the endoscope insertion section, and are led to the proximal end side of the endoscope are connected to the actuator serving as the drive section.

This manipulator is on the slave side, and performs an operation in accordance with the manipulation of the manipulation section of the master side arranged outside.

In general, a curving operation of the manipulator has an operation amount corresponding to a manipulation amount of the manipulation section. However, as a system configured in consideration of the individual character of the operator, there is, for example, a system equipped with an electric curving mechanism described in Jpn. Pat. Appln. KOKAI Publication No. 8-071072.

In this system, a drive signal is generated by adjusting an actual manipulation amount of the manipulation section by using a fixed control parameter fitted to the individual character of the operator, and an electric curving operation conformed to the drive signal is realized. That is, a movement amount and a moving speed of the manipulator is controlled in consideration of the personal habit and the degree of proficiency of the operator, thereby realizing further improvement in the degree of safety and the operability.

When the manipulator of the therapeutic device is used for a soft endoscope apparatus, it is used for an insertion part having flexibility and inserted into the therapeutic device hole of the insertion section of the endoscope, and a curving section of the multiarticular structure having a high degree of freedom provided on the distal end side. In the curving section of the multiarticular structure, the articulation section is curved by the traction of the wires.

Accordingly, the positional posture of an end effector (for example, a therapeutic device) provided at the distal end of the curving section are determined by the articulation parameter (in this case, an angle formed between articulations) of each articulation section.

Accordingly, an inverse problem for obtaining a target value of the articulation parameter for coinciding the positional posture of the end effector with the target positional posture of the operator is solved and, thereafter, drive control is performed in such a manner that the current articulation parameter value coincides with the target parameter value. As described above, in the therapeutic device, the insertion section and the curving section are curved in accordance with the shape inside the body cavity of the patient, and hence the operation is performed in consideration of the curved state of the insertion section.

An embodiment according to the present invention provides a therapeutic device system and a manipulator system which detect a curved state of an endoscope insertion section, adjust an operation of a therapeutic device inserted into the insertion section and an operation of a manipulator in accordance with the curved state, and operate smoothly with excellent operability.

Further, an embodiment according to the present invention provides a therapeutic device system comprising: an input section for generating a manipulation signal from a manipulation-designated amount designated by manipulation section; any one of a therapeutic device and a manipulator which includes articulation sections, a therapeutic device insertion section of which is inserted into a therapeutic device hole formed through to any one of an endoscope insertion section and an overtube attached to the outside of an endoscope, and in which the articulation sections can perform an articular operation in accordance with a manipulation instruction of the input section; a detection section for detecting a curved state of the curving section at all times; a therapeutic device drive section for driving the therapeutic device in accordance with the manipulation signal; and a control section provided with a control parameter set in advance, for controlling the articular operation of the articulation sections by changing the control parameter at all times on the basis of a detection result from the detection section, and causing the therapeutic device drive section to make the manipulation signal reflect the changed control parameter.

Further, an embodiment according to the present invention provides a manipulator system comprising: a distal end section formed into an external shape which can be inserted into an insertion path with a predetermined diameter;

a first movable section formed into an external shape which can be inserted into the insertion path, and connected to the distal end section; a first articulation section provided at a connection section of the distal end section and the first movable section, for connecting the distal end section and the first movable section to each other so that the section and the section can move relatively to each other; a second movable section formed into an external shape which can be inserted into the insertion path, and connected to the first movable section; a second articulation section provided at a connection section of the first movable section and the second movable section, for connecting the first movable section and the second movable section to each other so that the section and the section can move relatively to each other; a manipulation section capable of arbitrarily manipulating a manipulator section constituted of the distal end section, the first articulation section, the first movable section, the second articulation section, and the second movable section; a detection section which can detect external force exerted on the manipulator section from the insertion path; and a control section for controlling a drive state of the manipulator section on the basis of a detection result from the detection section.

The invention can be more fully understood from the following detailed description when taken in conjunction with the accompanying drawings, in which:

FIG. 1 is a block diagram showing the configuration of a therapeutic device system.
FIGS. 2A and 2B are views each showing the specific configuration of a multiarticular manipulator of this embodiment.
FIG. 3 is a view showing an example of a multiarticular structure model of the manipulator of this embodiment.

An embodiment of the present invention will be described below in detail with reference to the accompanying drawings.

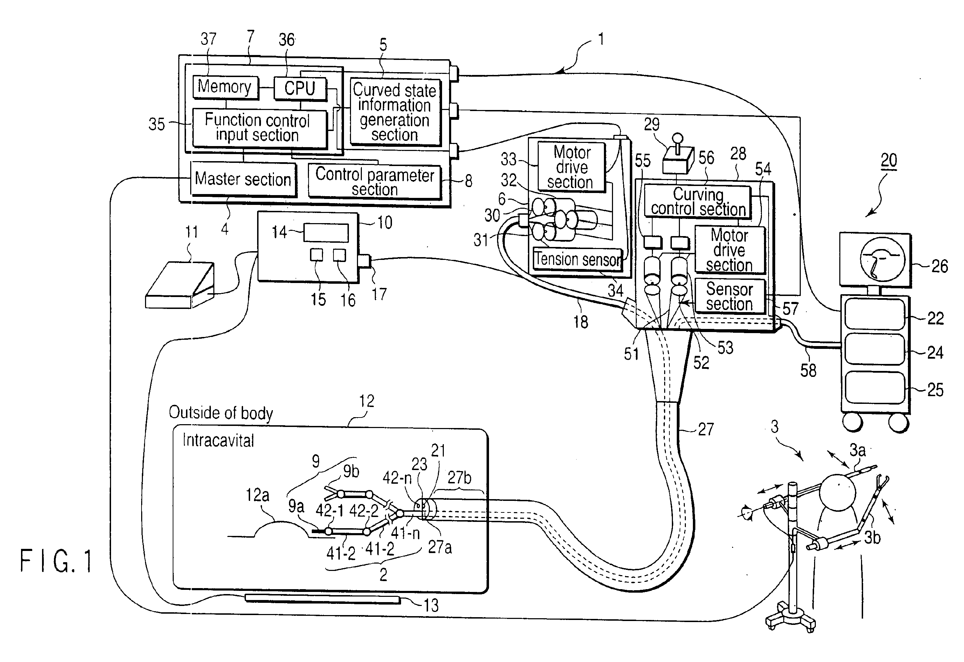

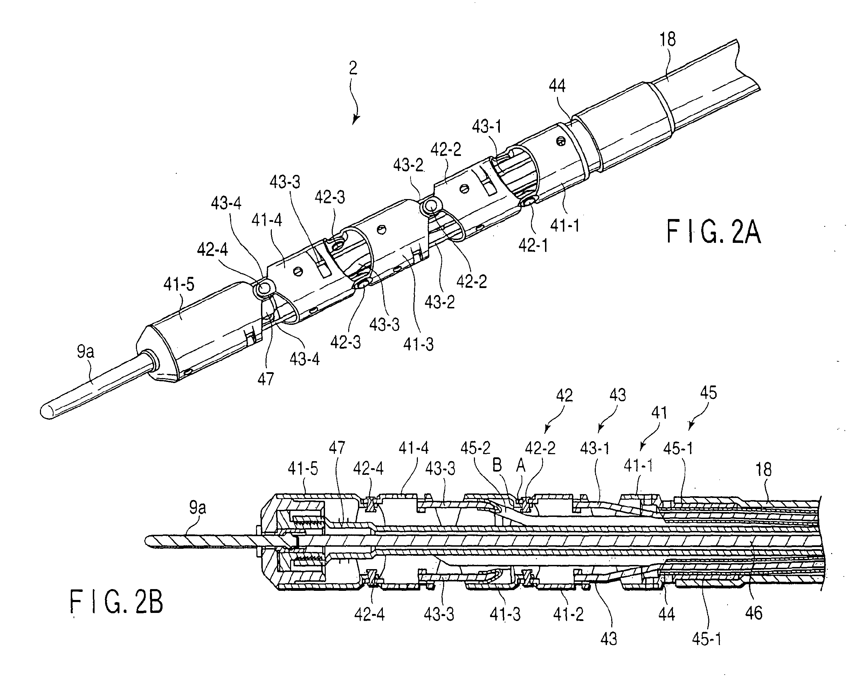

A therapeutic device system and a manipulator system of a first embodiment according to the present invention will be described below. FIG. 1 is a block diagram showing the configuration of the therapeutic device system. FIGS. 2A and 2B are views each showing the specific configuration of a multiarticular manipulator 2 used in the therapeutic device or the like of this embodiment.

In the therapeutic device system of this embodiment, a therapeutic device and/or a manipulator inserted into an insertion section of an endoscope apparatus generate or generates, with respect to a change in force exerted in a state where the insertion section of the endoscope apparatus is curved, a control parameter corresponding to the curved state, and the control parameter is added to a drive signal for driving the therapeutic device and the manipulator, whereby an articular operation is performed at a position or an angle desired by the operator.

The therapeutic device system 1 is inserted into a therapeutic device hole (forceps channel hole) of an endoscope insertion section 27 of an endoscope apparatus 20, or a therapeutic device hole of an overtube attached to the outside of the endoscope. The therapeutic device system 1 is a master-slave type electric therapeutic device for causing a therapeutic device and a manipulator extending from a distal end a therapeutic device hole thereof to perform a curving operation and a treating operation by means of, for example, an articulation section and wire traction.

Incidentally, it is assumed that endoscopes and therapeutic devices described in the following embodiments are active endoscopes and active therapeutic devices for performing operations such as a curving operation of the articulation section, and operations such as opening/closing, grasping, and the like at the movable part of the therapeutic device by means of a power source such as a motor and the like. Further, the above endoscopes and therapeutic devices are simply called endoscopes and therapeutic devices in the following description. Further, in addition to the electric drive source for driving by the magnetic force such as that of a motor or the like, a hydraulic drive source, and a pneumatic pressure drive source also belong to the category of the power source.

This therapeutic device system 1 is roughly comprised of a therapeutic device insertion section 18 which is inserted into a therapeutic device hole of an endoscope insertion section 27, can be advanced or retreated, and can be freely curved, a manipulator 2 provided at a distal end of the therapeutic device insertion section 18, and extending outwardly from a therapeutic device hole opening 27a of the endoscope insertion section 27, a manipulation section 3 by which the operator performs an operation instruction, a master section 4 for generating a manipulation signal corresponding to a manipulation amount of the manipulation section 3, a curved state information generation section 5 for generating curved state information to be described later, a manipulator drive section (actuator) 6 for driving the manipulator 2, a therapeutic device control section 7 for generating a control signal by adjusting the manipulation signal from the master section 4 by using a control parameter, and drive-controlling the manipulator drive section 6, and a control parameter section 8 for calculating a control parameter for adjusting the drive amount of the manipulator with respect to the manipulation signal on the basis of the curved state information, and providing the calculated control parameter to the therapeutic device control section 7.

Incidentally, in this embodiment, a curving operation of the articulation section of each of the therapeutic device and the manipulator inserted into the soft endoscope insertion section will be described below as an example. However, in these articulation sections, some of them perform not only a curving operation, but also, for example, an opening/closing operation of a linear-motion therapeutic device. The curving operation can also be easily applied to these articulation sections.

Incidentally, in this embodiment, the master section 4, curved state information generation section 5, therapeutic device control section 7, and control parameter section 8 are contained in a housing, and function as a control section of the therapeutic device system 1. Further, in the following embodiment, an articular operation of the manipulator 2 will be mainly described as an example.

In this embodiment, an example in which an electric-cautery 9a and a grasping instrument (for example, a forceps) 9b are provided as a therapeutic device unit is shown. Further, the electric-cautery 9a is used, and hence a power supply device 10 for supplying high-frequency power to the electric-cautery 9a, a foot switch 11 for issuing an instruction to supply high-frequency power to the high-frequency electric-cautery by a foot operation of the operator, and a counter electrode plate 13 connected to the power supply device 10, and attached to a body surface of a patient 12 to be subjected to a treatment are further provided. Incidentally, as for the therapeutic device, general therapeutic devices, or a combination of these may be provided in addition to the electric-cautery 9a and the forceps, and the like.

The power supply device 10 is provided with a display 14 for displaying a supply state and the like of power, an output wattage input panel 15, an output mode selection panel 16, and a power output terminal 17. The power output terminal 17 supplies high-frequency power output from a power unit (not shown) provided inside to the electric-cautery 9a.

The endoscope apparatus 20 is constituted of an image processing section 22 for subjecting image data taken by an image pickup section 21 provided at a distal end of the endoscope insertion section 27 to various image processing and data processing, a light source section 24 arranged adjacent to the image pickup section 21, for generating illumination light for illuminating an observation visual field including a lesioned part 12a from an illumination light window 23 through a light guide fiber (not shown), an endoscope control section 25 for performing control of the entire endoscope apparatus system, arithmetic processing, and the like, a monitor 26 for displaying a taken image, data relating to the image, an apparatus state, a manipulation instruction, and the like, an endoscope insertion section 27 a distal end part of which is provided with a multiarticular mechanism equivalent to the manipulator 2, and which is provided with an endoscope curving section 27b that can be curved, an electric-powered curving manipulation section 28 for curving the endoscope curving section 27b by means of electric power, and a curving joystick 29 for instructing to perform a curving manipulation of the endoscope.

The electric-powered curving manipulation section 28 has a configuration substantially equivalent to the manipulator drive section 6 to be described later, and includes a plurality of wires 51 for traction, a plurality of pulleys 52 each of which is connected to the other end of each wire 51, motors 53 rotation shafts of which are each fitted with the pulleys 52, a motor drive section 54 for individually driving each motor 53, encoders 55 with which the motor 53 are provided, a curving control section 56 for controlling the motor drive section 54 on the basis of values detected by the encoders 55, and a sensor section 57 including tension sensors for detecting values of tension exerted on the wires 51, and a strain gauge for detecting a strain amount of the endoscope insertion section 27. Further, the curving control section 56 is connected to the curving joystick 29, and an instruction of the curving manipulation is input thereto. Further, the curved state information generation section 5 generates curved state information from pulley angular information (wire traction information) of the pulley 52 detected by the encoder 55 functioning as a sensor section.

Further, the electric-powered curving manipulation section 28 is connected to the apparatus main body 20 with a cable 58. This cable includes the light guide fiber for sending illumination light, and signal lines constituted of an image signal line, a control signal line, and the like. Further, in this embodiment, the configuration example in which each of the endoscope and the therapeutic device is provided with a joystick is shown. However, the configuration may be made in such a manner that these manipulation functions are integrated into one joystick. Further, the example in which the therapeutic device system of this embodiment is applied to the endoscope apparatus configured in such a manner that the electric-powered curving manipulation section 28 and the endoscope insertion section 27 are fixedly connected to each other is described. However, it is possible to also apply the therapeutic device system of this embodiment to an endoscope apparatus so configured as to allow the endoscope insertion section to be attachable/detachable to/from the electric-powered curving manipulation section. Incidentally, in the case of a configuration in which a plurality of endoscope insertion sections can be selectively connected to one electric-powered curving manipulation section in turn, it is sufficient if a specific control parameter is registered in advance in a table of a memory provided in the control section for each endoscope insertion section, and a control parameter corresponding to the endoscope insertion section is read and set when the endoscope insertion section is connected to the electric-powered curving manipulation section. Further, it is desirable that a sensor and the like for detecting the curved state of the endoscope insertion section be provided as in this embodiment.

The therapeutic device system of this embodiment will be described below in detail.

First, the configuration of the manipulator 2 of the embodiment will be described below. FIG. 2A shows an example of the external appearance configuration of the manipulator 2, and FIG. 2B shows an example of the cross-sectional configuration of the manipulator 2.

This manipulator 2 includes a plurality of cylindrical curving pieces 41 (41-1, 41-2, 41-3, 41-4, and 41-5), axle members 42 (42-1, 42-2, 42-3, and 42-4) for coupling these curving pieces 41 so that they can be freely bent and curved, an electric-cautery 9a (or a grasping section 9b) provided on the curving piece 41-1 on the distal end side of the manipulator 2, a therapeutic device insertion section 18 which can be bent or curved in a comparatively soft and elastic manner, and a coupling member 44 for coupling the therapeutic device insertion section 18 and the curving piece 41-1 on the proximal end side to each other.

The inside of the linked structure of these curving pieces 41 and the axle members 42 is constituted of wires 43 (43-1, 43-2, 43-3, and 43-4) each of which is fixed to each of the curving pieces 41 at a distal end part by brazing or the like, and in which at least two wires are paired, flexible coils 45 (45-1, 45-2, 45-3, and 45-4) through which the wires are respectively passed, and which are provided between the respective curving pieces 41 and the connector of the actuator 6 in a penetrating manner, a power supply line 46 for supplying high-frequency power to the electric-cautery 9a, and a flexible tube provided in such a manner that the power supply line 46 is passed therethrough from the proximal end side of the endoscope insertion section 27 to the curving piece 41-5 at the distal end of the manipulator 2.

The linked structure of the curving pieces 41 and the axle members 42 will be described below.

Each of the curving pieces 41 excluding the curving pieces arranged at the distal end and the trunk end (proximal end side) is provided with two distal end side protrusion sections each having a tongue-like shape provided on the distal end side thereof with the central axis of the cylindrical curving piece interposed between them, and two tongue-like proximal end side protrusion sections provided on the proximal end side thereof with the central axis of the cylindrical curving piece interposed between them in a direction perpendicular (90° rotation) to the distal end side protrusion sections.

The linkage configuration between adjacent curving pieces 41 is such that as shown in FIG. 2A, for example, a hole is formed in each of the proximal end side protrusion section A of the curving piece 41-2, and the distal end side protrusion section B of the curving piece 41-3, the holes are caused to overlap each other, and the rivet-like axle member 42-2 is inserted into the holes, and thus the curving pieces 41-2 and 41-3 are linked with each other so that they can be swung, i.e., they can be curved freely. In this manner, the curving pieces 41 are universal-joint-connected to each other by means of the axle members 42, thereby forming a plurality of linked stages. By the linkage described above, in the linkage structure, each curving piece 41 is shifted 90° from the curving pieces adjacent in front and behind in posture.

In the universal-joint-connected linkage structure in which the connection positions of the axle members 42 between the adjacent curving pieces 41 are alternately shifted 90° as described above, by pulling one of the wires 43 of a desired curving piece 41, the curving piece is swung around two axle members 42. Accordingly, it is possible to perform a so-called articular operation in which two curving pieces 41 are freely curved or stretched straight according to the degree of the traction of the wire 43 of the desired curving piece 41, and move the electric-cautery 9a or the grasping section 9b three-dimensionally to a desired position.

The manipulator drive section 6 drives the manipulator 2 by means of electric power in accordance with a control signal from the therapeutic device control section 7. The manipulator drive section 6 is constituted of a plurality of wires 30 each of which has one end connected to a curving piece 41 of the manipulator 2, a plurality of pulleys 31 each of which is connected to the other end of each of the wires 30, motors 32 each of which serves as the drive source of the wire traction, rotation shafts of which are each fitted with the pulleys 31, a motor drive section 33 for individually driving each motor 32, and tension sensors 34 for detecting tension of the wires 30.

The manipulation section 3 is constituted of arm manipulation sections 3a and 3b each having a simplified multiarticular arm mechanism as shown in FIG. 1. As a sensor for detecting the arm manipulation, a magnetic sensor or an acceleration sensor is used, and an operation amount and an operation direction of each curving piece of the arm mechanism are detected. As the other sensor, a light emission source (for example, a laser light emitting element) is provided in each curving piece, and a light receiving element is arranged on a fixed member such as a support. It is also possible to detect the operation amount and the movement direction by an incident angle, signal strength (degree of attenuation), and the like of light incident on the light receiving element.

A manipulation signal based on a movement amount (manipulation instruction amount) of the arm manipulation sections 3a and 3b manipulated by the user is generated by the master section 4, and is output to the therapeutic device control section 7. In the manipulation section 3, as general input parts other than the arm mechanism, for example, a button switch, a joystick, a keyboard, and the like can be used. The manipulation instruction issued from the manipulation section 3 has a master-servant relationship with respect to the manipulator 2, and the manipulator 2 serving as the servant executes a curving operation or treatment in accordance with the manipulation instruction issued from the manipulation section 3 which is the master. Further, the manipulator 2 is inserted into the body cavity, and is remotely controlled, and hence the therapeutic device cannot be visually confirmed in a direct manner. Accordingly, the manipulation section 3 is manipulated while a dynamic picture image taken by the endoscope is viewed on the monitor 26, thereby sending an instruction from the master section 4.

As for the curved state of manipulator 2 of the therapeutic device, the positional posture thereof can be found from the tension value of the tension sensor 34. Here, the curving operation of the manipulator 2 will be described below.

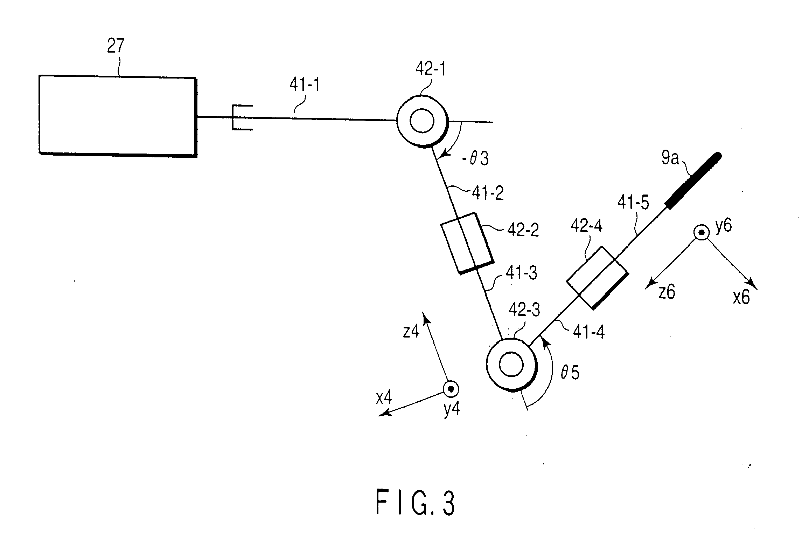

FIG. 3 shows a multiarticular structure model having four degrees of freedom provided by the five curving pieces 41-1 to 41-5, and the four articulation sections 42-1 to 42-4 of the manipulator 2 of this embodiment. Incidentally, although not shown in FIG. 3, on the proximal end side of a normal manipulator 2, axle members for curving the entire manipulator 2 in the axial direction, and axle members for moving the entire manipulator 2 in the direction around the axis are provided. The four axle members 42-1 to 42-4 curve the curving pieces alternately in the axial direction and in the direction around the axis. In the configuration described above, in, for example, FIG. 3, the state where both the axle member 42-1 and the axle member 42-3 are turned, and the part between the curving piece 41-1 and the curving piece 41-2, and the part between the curving piece 41-3 and the curving piece 41-4 are bent is shown. The current positional posture of the multiarticular manipulator 2 is detected. When the articulation angle (an articulation parameter which is one of so-called control parameters) of the axle members 18 is expressed by the following expression (1),

$$\Phi = \left(\theta_1, \theta_2, \cdots, \theta_n\right)^T \tag{1}$$

as shown in, for example, FIG. 3, the folding angle of the axle member 42-1 becomes $-\theta_3$, and the folding angle of the axle member 42-3 becomes $\theta_5$. As for the folding angles of the axle members 42 other than these, the articulation parameters remain 0 as long as they do not change from the initial positions. The positional posture of the electric-cautery 9a can be expressed by the following expression (2) as follows.

$$E_p = \left(x_{Ep}, y_{Ep}, z_{Ep}, Roll_{Ep}, Yaw_{Ep}, Pitch_{Ep}\right)^T \tag{2}$$

The relationship can be expressed by the following expression (3) as follows.

$$E_p = A(\Phi) \tag{3}$$

Here, the current positional posture of the electric-cautery 9a is expressed by the following expression (4) as follows.

$$E_{pnow} = \left(x_{Epnow}, y_{Epnow}, z_{Epnow}, Roll_{Epnow}, Yaw_{Epnow}, Pitch_{Epnow}\right)^T \tag{4}$$

The target positional posture to which the electric-cautery 9a is moved is set by the following expression (5) as follows.

$$P_p = \left(x_{Pp}, y_{Pp}, z_{Pp}, Roll_{Pp}, Yaw_{Pp}, Pitch_{Pp}\right)^T \tag{5}$$

Then, in order to bring the electric-cautery 9a into the state of the target position Pp, it is necessary to change the articulation parameter $\Phi$ from $\Phi$ satisfying the following expression (6)

$$E_{pnow} = A\left(\Phi_{now}\right) \tag{6}$$

to $\Phi$ satisfying the following expression (7).

$$P_p = A\left(\Phi_P\right) \tag{7}$$

These relational expressions are nonlinear, and hence, in order to obtain Φp, the Jacobian determinant J(Φ) obtained by subjecting Ep to partial differentiation by using the element of Φ is obtained as follows.

$$J(\Phi) = \begin{pmatrix} dx_{ep}/d\theta_1 & dx_{ep}/d\theta_2 & \cdots & dx_{ep}/d\theta_n \\ dy_{ep}/d\theta_1 & dy_{ep}/d\theta_2 & \cdots & dy_{ep}/d\theta_n \\ dz_{ep}/d\theta_1 & dz_{ep}/d\theta_2 & \cdots & dz_{ep}/d\theta_n \\ dRoll_{ep}/d\theta_1 & dRoll_{ep}/d\theta_2 & \cdots & dRoll_{ep}/d\theta_n \\ dYaw_{ep}/d\theta_1 & dYaw_{ep}/d\theta_2 & \cdots & dYaw_{ep}/d\theta_n \\ dPitch_{ep}/d\theta_1 & dPitch_{ep}/d\theta_2 & \cdots & dPitch_{ep}/d\theta_n \end{pmatrix} \tag{8}$$

From the following expression (9),

$$\dot{\Phi} = J(\Phi)^{-1}\dot{E}_p \tag{9}$$

Φ p satisfying the following expression (10)

$$P_p = A(\Phi_P) \tag{10}$$

$$\alpha = (a_1, a_2, \cdots, a_n) \tag{11}$$

can be obtained by convergent calculation. These calculation operations, i.e., calculation of the target positional posture is operation-processed by a CPU 36 in the therapeutic device control section 7.

The curved state information generation section 5 will be described below.

The curved state information generation section 5 generates curved state information (curved attitude position) on the endoscope insertion section 27 and the curving section 27b from the detection value of the sensor section 57.

Here, the curved state information is constituted of at least curved state information on the endoscope insertion section 27, curved state information on the curving section 27b provided on the distal end side of the endoscope insertion section 27, and the rotation amount of the pulley 52, or the wire length of the wire 51 pulled by the rotation amount of the pulley 52, or curved state information on the paid-out wire of the paid-out length.

Of these pieces of information, the curved state information on the insertion section 27 is information on the strain amount of the insertion section 27 detected by using a strain gauge of the sensor section 57. It is possible to estimate the current curved state of the insertion section 27 from this strain amount. Incidentally, it is also possible to detect a change in the curved state of the insertion section 27 by using the tension sensor of the sensor section 57. By assuming that the tension value of the tension sensor in the state where the curving section 27b is linear (i.e., in the state where no load is imposed on the curving wire of the therapeutic device) to be the initial value, it is possible to find the degree of the curved state of the curving section 27b from a change in the tension value.

Next, the control parameter section 8 will be described below.

First, the necessity of changing the control parameter will be described below.

In this embodiment, at the time of treating an affected part by using a therapeutic device, the manipulator 2 is driven to be curved by the traction of the wires performed by using the motors as drive sources, and the therapeutic device is set to a desired position of the affected part. The therapeutic device insertion section 18 is passed through the endoscope insertion section 27 and the curving section 27b which are curved in accordance with the shape inside the body cavity of the patient. Accordingly, the smaller the arc of the curvature is or the more the number of curved parts of the endoscope insertion section 27 or the curving section 27b is, the more the path length of the wires 30 arranged inside the therapeutic device insertion section 18 is changed, or the more the load is changed to be imposed.

As described previously, even when the convenience of manipulation is provided by using a control parameter (fixed value such as the articulation parameter or the like) set in advance, a situation different from the movement (the moving speed and the degree of the curved state) assumed by the operator occurs due to the load changing in accordance with the curved state.

In this embodiment, the control parameter is changed each time the change amount (signal value) of the curved information determined in advance is exceeded. That is, each time a certain change in the curved state occurs, the control parameter is changed (rewritten) in accordance with the curved state in the insertion section and the curving section, whereby even when the curved state is changed, a movement (the moving speed or the degree of curvature) of the manipulator or the therapeutic device conforming to the manipulation of the operator is performed. Incidentally, changing the control parameter may be performed not only in accordance with the change amount of the curved state but also on the basis of a predetermined period of time. The changing of the control parameter is continuously performed as long as the therapeutic device or the manipulator 2 is driven. That is, during the drive, the control parameter is changed at all times.

The control parameter section 8 of this embodiment stores therein a control parameter for adjusting the manipulation signal with respect to the drive amount of the manipulator 2 on the basis of the curved state information, concomitantly with a change in the curved state, calculates the control parameter as required in accordance with an operational expression or a program set in advance, and outputs the calculated control parameter to a corresponding table of a control table provided in the therapeutic device control section 7 as required, thereby updating the table.

The therapeutic device control section 7 is constituted of a function control input section 35 for inputting a manipulation instruction from the master section 4, and a control condition of a function or a control parameter from the control parameter section, a central processing unit (CPU) 36 for performing various operation processing, and instruction to each constituent section, and a memory 37 for storing images, communication data, and the like. The CPU 36 detects the positional posture (including the curved state) of the manipulator 2, and the operation state of the therapeutic device 9 by means of the detection signal of the tension sensor 34. In the memory 37, initial data at the operation start-up time, and an ID parameter (individual input ratio) for setting an operation condition for each selectable operator are stored. The ID parameter is a parameter for adjustment that enables standard manipulation or proper manipulation to be obtained while eliminating the personal habit of the operator in the manipulation.

The control parameters include teaching data of a slave manipulator for ovservation and treatment, a master-slave scale ratio, sensitivity, and the like. Of these parameters, the master-slave scale ratio is a parameter for determining how the operation amount of the manipulator 2 should be with respect to the operation amount of the arm of the manipulation section 3. In the case where the operation amount of the arm is the movement distance of the therapeutic device, and when the master-slave scale ratio is set at, for example, 1, and the movement amount of the distal end of the manipulation section 3 is 10 mm, the manipulator 2 operates in such a manner that the movement amount of the therapeutic device becomes 10 mm. On the other hand, when the master-slave scale ratio is 0.1, and when the movement amount of the distal end of the manipulation section 3 is 10 mm, the manipulator 2 operates in such a manner that the movement amount of the therapeutic device becomes 1 mm. In this example, although the scale ratio is that for positional movement, the scale ratio also applies to the angle ratio between the master side and the slave side.

Further, in the case where the sensitivity is used as the control parameter, for example, when a magnetic sensor is attached to the arm of the manipulation section 3, and the sensor signal is used as the curved state information, by changing the input sensitivity, the width of the dead band of the manipulator 2 can be changed. For example, when the input sensitivity is set at 1 mm, the manipulator 2 does not operate as long as the magnetic sensor does not move 1 mm or more. This makes it possible to eliminate a useless operation of the manipulator resulting from a shake or wobble of the operator's hand.

Next, an example of a change (rewriting) of the control parameter concomitant with calculation of a control parameter using curved state information, and a change in the curved state information will be described below.

For example, when the curved state of the endoscope is set as $\varepsilon$, the following are defined.

no curved state (straight, and not curved) $\varepsilon = 0$

a curved state on the positive side (upwardly curved when, for example, the horizontal direction is set as 0) $\varepsilon > 0$

a curved state on the negative side (downwardly curved when, for example, the horizontal direction is set as 0) $\varepsilon < 0$

Further, as for the lateral direction, judgment may be made likewise by setting the forward direction as 0, the right direction as the positive side, and the left side as the negative side. Needless to say, such setting may be appropriately determined at the time of setting.

Here, when there is no curved state ( ε = 0), if the motor target angle input and set by the operator is set as θ1, and the motor target angle determined by the detected curved state ε is set as θ2, θ2 is obtained from the following function.

$$\theta 2 \; = \; F(\theta 1, \; \varepsilon)$$

The function F(θ1, ε) is, for example, the following.

$$F(\theta 1, \; \varepsilon) \; = \; \theta 1 \; + \; D\varepsilon$$

(D: constant)

As described above, according to the therapeutic device system of this embodiment, the control parameter for adjusting the relationship between the amount of manipulation made by the operator, and the operation amount of the manipulator provided with the therapeutic device is changed in accordance with the curved state of the endoscope insertion section. Even when the curved state of the endoscope insertion section is changed as a result of this change, the control parameter in the changed state is calculated, and the previous control parameter is rewritten. Accordingly, it is possible for the operator to operate the manipulator by the same manipulation operation at all times. Therefore, unlike the conventional case, the operability of the manipulator becomes better without being affected by the curved state of the endoscope insertion section, and the labor required to perform the treatment is reduced.

Next, the drive control of the therapeutic device system performed by the therapeutic device control section 7 will be described below.

First, the therapeutic device control section 7 is subjected to initialization processing at the time of turn-on and start-up. At this time, teaching data set in advance is set as the initial data. Hereine, when there is an ID parameter set in accordance with the individuality of the operator in the manipulation, the ID parameter is read from the memory file, and condition-setting is performed. At the same time, as for the proper control parameter owned by each therapeutic device insertion section 18 of the endoscope apparatus to be used, the operator inputs or registers in advance a control parameter owned by each therapeutic device to be used in a table of a memory (not shown) in the control parameter section 8, and a control parameter is selected and read from the parameter group, whereby the initial setting is performed.

The operator (operating surgeon) grasps the manipulation section 3, and performs an operation while viewing the monitor 26. In accordance with the movement of the hands of the operator, a signal indicating the manipulation-designated amount is input from the arm manipulation section 3a or 3b to the master section 4. The master section 4 generates a manipulation signal from the input signal, and outputs the manipulation signal to the function control input section 35.

Further, curved state information is generated from the curved state of the endoscope insertion section 27 detected by the sensor section 57 and the encoder 55, and is output to the function control input section 35. Likewise, a detection signal is output from the tension sensor 34 to the function control input section 35. The function control input section 35 outputs the manipulation signal, the curved state information, and the control parameter to the CPU 36. In the CPU 36, operation processing is performed by the operation method described previously, control signals based on the operation result are output to the respective motor drive sections 33 and 54, and the respective articulation sections constituted of the respective curving pieces 41 are caused to perform an articular operation (bending or linear extension), whereby the therapeutic device is moved to a position desired by the operator.

As has been described above, in the therapeutic device system of this embodiment, the therapeutic device and/or the manipulator inserted into the insertion section of the endoscope apparatus generate or generates, with respect to a change in force exerted in a state where the insertion section of the endoscope apparatus is curved, a control parameter corresponding to the curved state, and the drive signal at the therapeutic device or the manipulator is adjusted so as to adjust the operation, whereby an articular operation is performed at a position or an angle desired by the operator, and the operation can be performed with good operability and smoothly.

**Claims**

**1.** A manipulator system **characterized by** comprising:

a distal end section 41-5 formed into an external shape which can be inserted into an insertion path with a predetermined diameter;

a first movable section 41-4 formed into an external shape which can be inserted into the insertion path, and connected to the distal end section 41-5;

a first articulation section 42-4 provided at a connection section 43-4 of the distal end section 41-5 and the first movable section 41-4, for connecting the distal end section 41-5 and the first movable section 41-4 to each other so that the section 41-5 and the section 41-4 can move relatively to each other;

a second movable section 41-3 formed into an external shape which can be inserted into the insertion path, and connected to the first movable section 41-4;

a second articulation section 42-3 provided at a connection section 43-3 of the first movable section 41-4 and the second movable section 41-3, for connecting the first movable section 41-4 and the second movable section 41-3 to each other so that the section 41-4 and the section 41-3 can move relatively to each other;

a manipulation section 3 capable of arbitrarily manipulating a manipulator 2 constituted of the distal end section 41-5, the first articulation section 42-4, the first movable section 41-4, the second articulation section 42-3, and the second movable section;

a detection section 5 which can detect external force exerted on the manipulator 2 from the insertion path; and

a control section 7 for controlling a drive state of the manipulator 2 on the basis of a detection result from the detection section.

2. The manipulator system according to claim 1, **characterized in that**
the detection section 5 detects a value of tension of a tension sensor 34 provided on a wire connected to the first movable section 41-4 and the second movable section 41-3.

3. The manipulator system according to claim 1, **characterized in that**
a manipulation instruction given by the manipulation section 3 and the manipulator 2 have a master-slave relationship, and in accordance with the manipulation instruction given by the manipulation section 3 which is the master side, the manipulator 2 which is the slave side performs an articular operation and treatment.

F I G. 1

F I G. 2A

F I G. 2B

FIG.3

EP 2 213 221 A1

Europäisches
Patentamt

European
Patent Office

Office européen
des brevets

# EUROPEAN SEARCH REPORT

Application Number

EP 10 00 4087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 800 593 A1 (OLYMPUS CORP [JP]; OLYMPUS MEDICAL SYSTEMS CORP [JP]) 27 June 2007 (2007-06-27) * paragraphs [0069], [0 75], [0 77]; figure 2 * ----- | 1-3 | INV. A61B1/01 A61B1/005 |
| X | US 2004/193015 A1 (IKEDA YUICHI [JP] ET AL) 30 September 2004 (2004-09-30) | 1,2 | |
| Y | * paragraph [0204]; figures 19A,B * ----- | 3 | |
| Y | WO 2007/111571 A (NANYANG TECHNOLOGICAL UNIVERSITY) 4 October 2007 (2007-10-04) * see especially figures 1, 2, 4 and 5 and the description thereof * ----- | 3 | |

TECHNICAL FIELDS
SEARCHED      (IPC)

A61B

The present search report has been drawn up for all claims

| Place of search | Date of completion of the search | Examiner |
|---|---|---|
| Munich | 21 June 2010 | Fischer, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## ANNEX TO THE EUROPEAN SEARCH REPORT
## ON EUROPEAN PATENT APPLICATION NO.

EP 10 00 4087

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2010

| Patent document cited in search report | | Publication date | Patent family member(s) | | Publication date |
|---|---|---|---|---|---|
| EP 1800593 | A1 | 27-06-2007 | WO<br>JP<br>US | 2006035693 A1<br>2006116289 A<br>2007173694 A1 | 06-04-2006<br>11-05-2006<br>26-07-2007 |
| US 2004193015 | A1 | 30-09-2004 | JP<br>US | 2003230536 A<br>2007112255 A1 | 19-08-2003<br>17-05-2007 |
| WO 2007111571 | A | 04-10-2007 | NONE | | |

EPO FORM P0459

For more details about this annex : see Official Journal of the European Patent Office, No. 12/82

**EP 2 213 221 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8071072 A